# FASCICULE DE BREVET EUROPEEN

(11) **EP 2 313 490 B1**
(45) Date de publication et mention de la délivrance du brevet: **30.03.2016**
(21) Numéro de dépôt: 09769529.0
(22) Date de dépôt: 19.06.2009
(51) Int. Cl.: C12N 1/20, A01N 63/02, C12N 15/31, C12R 1/465

(54) **NOUVELLE SOUCHE STREPTOMYCES BETA-VULGARIS, FILTRAT DE CULTURE, COMPOSES ACTIFS DERIVES ET LEUR UTILISATION DANS LE TRAITEMENT DES PLANTES**
NEUER STREPTOMYCES BETA-VULGARIS STAMM, KULTURFILTRAT, ABGELEITETE WIRKSTOFFE UND VERWENDUNG DAVON BEI DER BEHANDLUNG VON PFLANZEN
NEW STREPTOMYCES BETA-VULGARIS STRAIN, CULTURE FILTRATE, DERIVED ACTIVE COMPOUNDS AND USE THEREOF IN THE TREATMENT OF PLANTS

(30) Priorité: 19.06.2008 FR 0854051
(43) Date de publication de la demande: 27.04.2011
(73) Titulaire: Agronutrition, 31390 Carbonne (FR); Institute National Polytechnique de Toulouse, B.P. 34 038 31029 Toulouse Cedex 4 (FR)
(72) Inventeur: LEBRIHI, Ahmed, 31450 Noueilles (FR); ERRAKHI, Rafik, Benguerir (MA); BARAKATE, Mustapha, Marrakech 40000 (MA)
(74) Mandataire: Cabinet BARRE LAFORGUE & associés
(86) Numéro de dépôt international: PCT/FR2009/051181
(87) Numéro de publication internationale: WO 2009/156688

(56) Documents cités:
- RAFIK ERRAKHI ET AL: "Evidences of biological control capacities of Streptomyces spp. against Sclerotium rolfsii responsible for damping-off disease in sugar beet (Beta vulgaris L.)" WORLD JOURNAL OF MICROBIOLOGY AND BIOTECHNOLOGY, KLUWER ACADEMIC PUBLISHERS, DO, vol. 23, no. 11, 6 mai 2007 (2007-05-06), pages 1503-1509, XP019557899 ISSN: 1573-0972
- DATABASE EMBL [Online] 19 décembre 2005 (2005-12-19), "Streptomyces sp. B-11 16S ribosomal RNA gene, partial sequence." XP002515196 extrait de EBI accession no. EMBL:DQ303238 Database accession no. DQ303238 cité dans la demande
- BARAKATE M ET AL: "Characterization of rhizospheric soil streptomycetes from Moroccan habitats and their antimicrobial activities" WORLD JOURNAL OF MICROBIOLOGY AND BIOTECHNOLOGY, vol. 18, no. 1, février 2002 (2002-02), pages 49-54, XP002443083 ISSN: 0959-3993
- OUHDOUCH YEDIR ET AL: "Actinomycetes of Moroccan habitats: Isolation and screening for antifungal activities" EUROPEAN JOURNAL OF SOIL BIOLOGY, vol. 37, no. 2, avril 2001 (2001-04), pages 69-74, XP002515052 ISSN: 1164-5563

### SEQUENCE LISTING

<110> AGRONUTRITION
   Institut National Polytechnique de Toulouse
<120> Nouvelle souche Streptomyces Bet a- vulgaris, ses antibiotiques dér i vés et leur utilisation dans le traitement des plantes
<130> INPACT 02
<160> 11
<170> PatentIn version 3.3
<210> 1
   <211> 1442
   <212> DNA
   <213> Streptomyces Beta-Vulgaris- ADNr 16S - SEQ n° 1
<400> 1
   t t aacacat g caagt cgaac gat gaagccg ct t cggt ggt ggat t agt gg cgaacgggt g 60
   agt aacacgt gggcaat ct g ccct gcact c t gggacaagc cct ggaaacg gggt ct aat a 120
   ccggat at ga ct accgat cg cat ggt t ggt ggt ggaaagc t ccggcggt g caggat gagc 180
   ccgcggccta tcagcttgtt ggtggggtga tggcctacca aggcgacgac gggtagccgg     240
   cctgagaggg cgaccggcca cactgggact gagacacggc ccagactcct acgggaggca     300
   gcagtgggga atattgcaca atgggcgaaa gcctgatgca gcgacgccgc gtgagggatg     360
   acggccttcg ggttgtaaac ctctttcagc agggaagaag cgagagtgac ggtacctgca     420
   gaagaagcgc cggctaacta cgtgccagca gccgcggtaa tacgtagggc gcgagcgttg     480
   t ccggaat t a t t gggcgt aa agagct cgt a ggcggct t gt cacgt cggat gt gaaagccc 540
   ggggct t aac cccgggt ct g cat t cgat ac gggcaggct a gagt t cggt a ggggagat cg 600
   gaattcctgg tgtagcggtg aaatgcgcag atatcaggag gaacaccggt ggcgaaggcg     660
   gatctctggg ccgatactga cgctgaggag cgaaagcgtg gggagcgaac aggattagat     720
   accct ggt ag t ccacgccgt aaacgt t ggg aact aggt gt gggcgacat t ccacgt cgt c 780
   cgtgccgcag ctaacgcatt aagttccccg cctggggagt acggccgcaa ggctaaaact     840
   caaaggaatt gacgggggcc cgcacaagcg gcggagcatg tggcttaatt cgacgcaacg     900
   cgaagaacct taccaaggct tgacatacac cggaaacggc cagagatggt cgcccccttg     960
   t ggt cggt gt acaggt ggt g cat ggct gt c gt cagct cgt gt cgt gagat gt t gggt t aa 1020
   gtcccgcaac gagcgcaacc cttgttctgt gttgccagca tgcctttcgg ggtgatgggg    1080
   act cacagga gact gccggg gt caact cgg aggaaggt gg ggacgacgt c aagt cat cat 1140
   gcccct t at g t ct t gggct g cacacgt gct acaat ggccg gt acaat gag ct gcgat acc 1200
   gtgaggtgga gcgaatctca aaaagccggt ctcagttcgg attggggtct gcaactcgac    1260
   cccat gaagt cggagt cgct agt aat cgca gat cagcat t gct gcggt ga at acgt t ccc 1320
   gggccttgta cacaccgccc gtcacgtcac gaaagtcggt aacacccgaa gccggtggcc    1380
   caaccccttg gggagggagc tgtcgaaggt gggactggcg attgggacga agtcgtaaca    1440
   ag 1442
<210> 2
   <211> 20
   <212> DNA
   <213> amorce universelle 27 f - SEQ n° 2
<400> 2
   agagt t t gat cct ggct cag 20
<210> 3
   <211> 19
   <212> DNA
   <213> amorce universelle 1492 r - SEQ n° 3
<400> 3
   ggt t acct t g t t acgact t 19
<210> 4
   <211> 24
   <212> DNA
   <213> amorce PR1 f - SEQ n° 4
<400> 4
   ctggctattc tcgattttta atcg 24
<210> 5
   <211> 24
   <212> DNA
   <213> amorce PR1 r - SEQ n° 5
<400> 5
   t cct gcat at gat gct cct t at t g 24
<210> 6
   <211> 30
   <212> DNA
   <213> amorce PDF1 f - SEQ n° 6
<400> 6
   t cat ggct aa gt t t gct t cc at cat caccc 30
<210> 7
   <211> 24
   <212> DNA
   <213> amorce PDF1 r - SEQ n° 7
<400> 7
   gt agat t t aa cat gggact g cgac 24
<210> 8
   <211> 21
   <212> DNA
   <213> amorce PAL1 f - SEQ n° 8
<400> 8
   cggaggagga gtggacgcta t 21
<210> 9
   <211> 21
   <212> DNA
   <213> amorce PAL1 r - SEQ n° 9
<400> 9
   tgcgacaccg tttttggttc t 21
<210> 10
   <211> 24
   <212> DNA
   <213> amorce EF1 f - SEQ n° 10
<400> 10
   at gccccagg acat cgt gat t t ca 24
<210> 11
   <211> 24
   <212> DNA
   <213> amorce EF1 r - SEQ n° 11
<400> 11
   t t ggcggcac cct t agct gg at ca 24

## Description

La présente invention a pour objet une nouvelle souche actinomycète Streptomyces beta-vulgaris ou une souche mutante de celle-ci.

L'invention vise aussi l'utilisation de cette souche pour produire une composition, ou filtrat de culture, par fermentation et des composés actifs.

L'invention concerne également l'utilisation de cette souche beta-vulgaris, d'une souche mutante, du filtrat de culture ou d'un composé actif pour stimuler la croissance des plantes et/ou pour lutter contre des infections causées par des agents phytopathogènes.

Depuis plusieurs décennies, le monde agricole cherche de nouvelles solutions pour améliorer la qualité des sols et pour stimuler la croissance végétale.

Dans ce but, de nombreux produits ont été développés pour fertiliser le sol, stimuler la nutrition et donc la croissance des plantes cultivées.

Toutefois, ces produits sont généralement essentiellement d'origine chimique et ne sont pas satisfaisants du point de vue environnemental et humain, si bien qu'il existe un besoin pour des substances d'origine naturelle capables de stimuler efficacement la croissance des végétaux.

De plus, les plantes sont fragiles et sont susceptibles d'être infectées par divers facteurs, en particulier par des agents phytopathogènes, qui réduisent de façon conséquente la qualité et le rendement de l'agriculture. Parmi les agents pathogènes, les champignons, responsables des maladies fongiques, sont ceux qui ont le plus grand impact économique. Chaque espèce végétale est sensible à une ou plusieurs maladies principales, susceptibles de réduire fortement sa vigueur, sa croissance, et finalement la quantité ou/et la qualité de la récolte.

On sait que les maladies phytogènes réduisent de 12 à 14% la production agricole mondiale, 70% des dommages étant d'origine fongique. Il est donc indispensable de trouver des moyens efficaces pour lutter contre les infections causées par des agents phytopathogènes.

Actuellement, plusieurs moyens de lutte sont employés.

Un premier moyen, simple à mettre en oeuvre, consiste à pratiquer des techniques culturales telles que le labourage ou la rotation des cultures. Toutefois, les agents pathogènes produisent des sclérotes dans le sol qui leur permettent de survivre pendant plusieurs années, si bien que les pratiques culturales restent inefficaces en absence de moyens éradiquant dans le sol les formes de résistance et le mycélium pathogène.

Une deuxième méthode, la plus utilisée, consiste à traiter chimiquement les cultures infectées. On connaît par exemple le traitement au bromure de méthyle, qui est le fongicide chimique le plus utilisé, généralement pour désinfecter le sol avant l'ensemencement des cultures.

Toutefois, le traitement chimique n'est pas non plus satisfaisant car l'utilisation de produits chimiques provoque des effets négatifs sur l'environnement et sur la santé humaine. Le bromure de méthyle en particulier constitue une menace pour la stratosphère à cause du pouvoir catalyseur de l'atome de brome.

En outre l'efficacité des agents chimiques est de plus en plus limitée du fait de l'apparition de souches pathogènes résistantes.

Depuis quelques années, des solutions plus efficaces et plus respectueuses de l'environnement ont donc été recherchées. Les spécialistes se sont orientés vers la lutte biologique basée sur l'utilisation d'agents biologiques antagonistes naturels.

Plusieurs microorganismes, tels que Trichoderma hazianum, Trichoderma koningii ou Pseudomonas fluorescens, ont été utilisés pour contrôler des maladies phytopathogènes.

Errakhi et al. (World J Microbiol Biotechnol 23 :1503-1509, 2007) décrit plusieurs souches de Streptomyces ayant des activités antagonistes contre des agents phytopathogènes.

Toutefois les agents biologiques existants ne sont pas satisfaisants en terme d'efficacité et de fiabilité et il subsiste un besoin pour des agents biologiques naturels non toxiques pour l'homme et l'environnement, capables de protéger efficacement les plantes en stimulant leur croissance et/ou en luttant contre les maladies phytopathogènes.

C'est pourquoi la présente invention vise à répondre à ce besoin en proposant une nouvelle souche bactérienne particulièrement efficace pour stimuler la croissance des végétaux et/ou lutter contre les infections phytopathogènes.

A cet effet l'invention a pour objet une souche actinomycète Streptomyces beta-vulgaris selon la revendication 1. La souche selon l'invention a été déposée à la Collection Nationale de Cultures de Microorganismes CNCM sous le numéro I-3639 et au centre de collection de microorganismes belge LGM sous le numéro P-23735. La séquence de l'ADNr 16S SEQ n°1 est déposée dans la banque de données de nucléotides Pubmed/NCBI, sous la référence : DQ303238.

Dans la description qui va suivre de l'invention, cette souche pourra être nommée « I-3639 ».

Avantageusement, cette souche est très efficace à la fois pour stimuler la croissance des plantes et pour lutter contre les agents phytopathogènes.

Avantageusement, cette souche est très efficace dans la lutte biologique contre les agents phytopathogènes. Elle est également efficace pour la stimulation de la croissance des plantes, en particulier en améliorant l'assimilation des nutriments par les plantes.

L'invention concerne également l'utilisation de la souche I-3639 ou d'une souche mutante, pour l'obtention par fermentation d'un filtrat de culture comprenant des métabolites secondaires, présentant une activité antibactérienne, antifongique et stimulatrice de la croissance des plantes.

La présente invention vise également l'utilisation de la souche I-3639 ou d'une souche mutante, pour l'obtention de métabolites ou composés actifs, en particulier des antibiotiques.

L'invention vise aussi l'utilisation de la souche I-3639 ou d'une souche mutante, du filtrat de culture obtenu par fermentation de la souche, ou des composés actifs obtenus par fermentation de la souche, pour le traitement des plantes, en particulier pour la lutte contre les infections phytopathogènes et/ou pour favoriser la croissance des plantes. En particulier il s'agit de l'utilisation :
- en tant qu'agent antibactérien pour la protection des plantes et la lutte biologique contre des agents phytopathogènes, par exemple pour la protection des plantes vis-à-vis de bactéries gram-positif, et/ou
- en tant qu'agent antifongique pour la protection des plantes et la lutte biologique contre des agents phytopathogènes, et/ou
- pour activer les systèmes de défense naturels des plantes, notamment pour stimuler l'expression des gènes de défense des plantes PR1 et PDF1.2a, et/ou
- pour stimuler la croissance des plantes, et/ou
- pour améliorer l'assimilation des nutriments par les plantes, notamment pour améliorer l'assimilation du Potassium, du Zinc, du Bore, du Calcium, du Cuivre, du Phosphore, du Magnésium, du Fer, du Manganèse et de l'Azote.

L'invention est maintenant décrite en détail, en regard des dessins annexés sur lesquels :

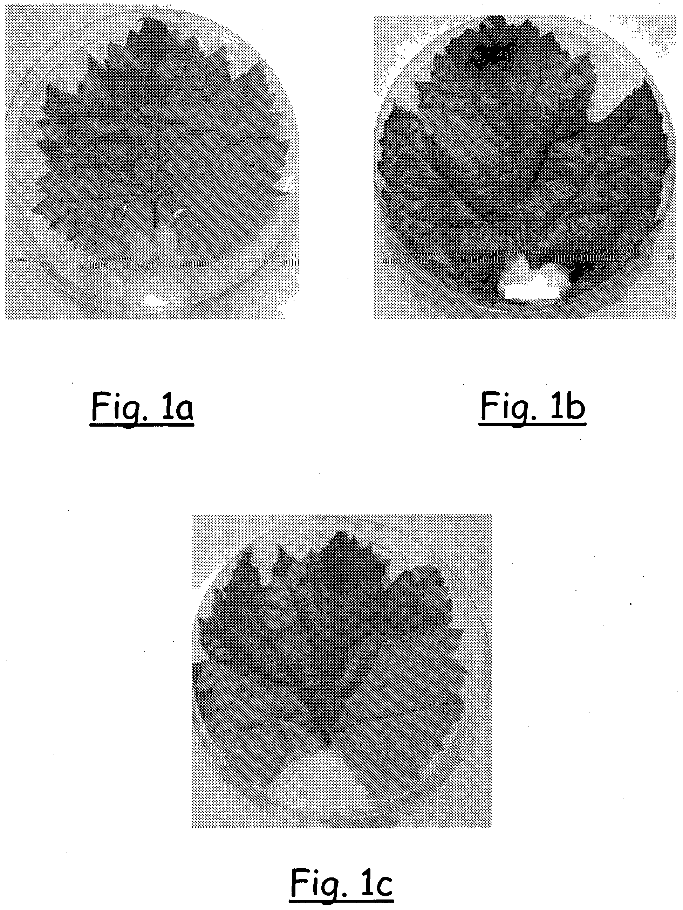

- la figure 1a représente une photo de feuille de vigne sans traitement (témoin),
- la figure 1b représente une photo de feuille de vigne traitée par Botrytis cinerea puis par le filtrat de culture obtenu par fermentation de la souche I-3639 selon l'invention, et
- la figure 1c représente une photo de feuille de vigne traitée par Botrytis cinerea.

### I. Protocole d'isolement de la souche selon l'invention

La souche beta-vulgaris I-3639 selon l'invention est isolée selon un procédé de sélection comprenant les étapes suivantes :
- mise en présence d'un échantillon biologique susceptible de contenir la souche beta-vulgaris et/ou une souche mutante, avec un milieu approprié pour la sélection de souches actinomycètes,
- traitement dudit échantillon pour isoler les souches actinomycètes présentes, et
- isolement de la souche beta-vulgaris et/ou d'une souche mutante, en fonction de sa capacité à inhiber des agents phytopathogènes.

L'isolement de la souche selon l'invention peut être effectué à partir d'échantillons du sol rhizosphérique de la betterave à sucre de Beni-Mellal au Maroc.

Le protocole est décrit en suivant.

Des échantillons de sol d'un sol rhizosphérique de la betterave à sucre de Beni-Mellal sont prélevés avec une tarière de plus de 10cm de profondeur, après avoir retiré environ 3cm de la surface du sol.

Les échantillons prélevés sont placés dans des sachets stériles en polyéthylène, fermés et stockés à 4°C.

Les souches actinomycètes sont ensuite isolées sur un milieu OL (Olson) ou un milieu SEA (Soil Extract Agar) selon deux méthodes : traitement par chaleur-agitation ou traitement par chimiotactisme.

Préférentiellement les échantillons sont traités par la méthode chaleur - agitation.

Les échantillons sont mélangés, mis en suspension dans de l'eau distillée stérile (4g pour 36ml) et agités à 200 tours par minute pendant 30 minutes. Ils sont ensuite traités à 50°C pendant 10 minutes.

Chaque échantillon est ensuite dilué à 10⁻⁷, dispersé (0,1mL) à la surface d'un milieu OL ou d'un milieu SEA, préférentiellement SEA, puis incubé à 30°C pendant 21 jours.

Selon une variante les échantillons peuvent être traités par chimiotactisme.

Des antibiotiques sont ajoutés dans le milieu d'isolation (OL et SEA), comme de l'acide nalixidique (10mg.L⁻¹) et de la novobiocine (25mg.L⁻¹). Préférentiellement on ajoute de l'acide nalixidique. Des composés antifongiques cycloheximides (40mg.L⁻¹) peuvent également être ajoutés au milieu.

Les échantillons sont ensuite incubés à 30°C pendant 3 semaines.

Le traitement des échantillons peut être précédé par une étape de pré-traitement par exemple par chauffage, micro-ondes, pulsions électriques ou encore à l'aide de réactifs chimiques.

Après traitement des échantillons on isole les souches actinomycètes par reconnaissance au microscope sur la base de leurs caractéristiques morphologiques.

Les actinomycètes sont transférés sur un milieu Bennett. Les isolats purs sont maintenus à 4°C pendant 2mois. Alternativement, les cultures sont resuspendues et conservées dans 20% de glycérol stérile à -20°C.

Pour isoler spécifiquement la souche objet de la présente invention, on réalise un criblage en fonction de sa capacité à inhiber les agents pathogènes suivants :
- Sclerotium rolfsii
- Fusarium oxysporum
- Verticillium dahliae
- Botrytis cinerae
- Pythium ultimum

La sélection est réalisée par diffusion agar avec la méthode des cylindres (Bauer et al., 1966). Des actinomycètes purs sont inoculés sur un milieu Bennett. Après une incubation de 5 jours à 30°C, un cylindre calibré (de 6 mm de diamètre) est découpé dans le milieu de culture et placé sur un milieu test contenant les agents phytopathogènes testés. Le milieu de culture est un milieu PDA pour les champignons pathogènes et un milieu Bennett pour les bactéries pathogènes. Les souches sont ensuite maintenues à 4°C pendant 4 heures pour permettre la diffusion des composés antibiotiques puis incubées à 30°C. Les diamètres d'inhibition sont déterminés après 48 heures d'incubation.

Parmi les différentes souches montrant une capacité d'inhibition des agents pathogènes testés, la souche I-3639 selon l'invention est sélectionnée pour sa capacité d'inhibition des sclérotes de Sclerotium rolfsii et de la croissance hyphale de Sclerotium rolfsii.

Pour le test de germination des sclérotes, des sclérotes de Sclerotium rolfsii sont collectés et désinfectés avec de l'hypochlorite de sodium à 2% pendant 3 minutes et lavés 3 fois avec de l'eau distillée stérile. Dix sclérotes ont été disposés à la surface d'un support agar contenant un milieu PDA mélangé avec les souches d'actinomycètes, et le nombre de sclérotes germés est compté après 72heures d'incubation à 25°C.

Pour le test de croissance hyphale, un disque mycélien de 8mm a été prélevé à partir de colonies de 5 jours de Sclerotium rolfsii et transféré sur un support. Les croissances mycéliennes sont évaluées en mesurant le diamètre des colonies après 72 heures d'incubation à 25°C.

La souche selon l'invention est sélectionnée pour sa capacité d'inhibition des sclérotes de Sclerotium rolfsii de 71% et de la croissance hyphale de Sclerotium rolfsii de 80%. Elle est reconnaissable par ces caractéristiques phénotypiques et physiologiques décrites ci-après.

### II. Caractérisation de la souche selon l'invention

La nouvelle souche beta-vulgaris objet de l'invention est une souche bactérienne appartenant à la famille des actinomycètes et au genre des Streptomyces. Elle été déposée le 30 juin 2006 à la CNCM sous le numéro I-3639 et au centre de collection des micro-organismes belge LGM sous le numéro P-23735.

Les actinomycètes ou les actinobactéries sont des bactéries à gram positif qui présentent une forme filamenteuse. Ce sont des bactéries saprophytes capables de dégrader de la matière organique dans le sol et d'utiliser des molécules plus complexes pour leur croissance.

La souche bactérienne beta-vulgaris selon l'invention est caractérisée par un mycélium aérien jaune blanchâtre sur milieu ISP2 ou Bennett gélose. Le mycélium de substrat varie de jaune brun à gris brun selon le milieu de culture.

La souche I-3639 présente des spores lisses à chaînes en spirales de type S. Les chaînes comprennent en moyenne 10 à 50 spores.

La température de croissance est comprise entre 12 et 37°C, préférentiellement entre 28 et 32°C. Le pH du milieu de culture est préférentiellement compris entre 5 et 9.

Pour sa croissance, la souche doit être mise en culture dans un milieu contenant une variété de substances nutritives. Par exemple, comme source de carbone on peut utiliser le D-glucose, le D-fructose, le D-maltose, le D-lactose, le L-rhamnose et la dextrine. En revanche, il est préférable de ne pas utiliser le L-arabinose, le raff inose, le sorbitol, le D-galactose et la cellulose.

Comme source d'azote on peut utiliser les acides aminés, les sels nitrate et les sels ammonium.

La souche selon l'invention n'hydrolyse pas l'amidon. Elle réduit les nitrates en nitrites et dégrade l'adénine, le Tween 20 et l'acétate de sodium.

La souche selon l'invention peut également être caractérisée par ses caractéristiques moléculaires. Les principales analyses moléculaires utilisées pour la détermination des espèces bactériennes sont le séquençage de l'ADN ribosomal (ADNr) 16S et l'hybridation ADN-ADN.

L'hybridation ADN-ADN montre que la souche I-3639 selon l'invention correspond à une nouvelle espèce dans le groupe des Streptomyces.

Le séquençage de l'ADNr 16S consiste à extraire de l'ADN génomique puis à amplifier par PCR la partie 16S ADNr en utilisant des amorces spécifiques. Le produit de la PCR est ensuite utilisé pour séquencer la partie du 16S ADNr. L'amplification de la partie ADNr 16S de la souche bactérienne selon l'invention a été effectuée en utilisant deux amorces universelles 27f de séquence SEQ n°2 (5'-*AGAG*TTT*GA*T*CC*T*GGC*T*CAG*-3') et 1492r de séquence SEQ n°3 (5'-*GG*TT*ACC*TTGTT*ACGAC*TT-3'). La séquence ADNr 16S caractérisant la souche bactérienne selon l'invention est la séquence SEQ n°1 :

Cette séquence SEQ n°1 est déposée dans la banque de donnée de nucléotides Pubmed/NCBI, sous la référence : DQ303238.

La présente invention vise également les souches mutantes de la souche beta-vulgaris I-3639.

Par « souche mutante », on entend désigner toute souche pouvant être obtenue par mutation sélective à partir de la souche beta-vulgaris I-3639.

Les souches mutantes selon l'invention sont obtenues en mettant la souche beta-vulgaris I-3639 en présence d'un agent mutagène physique, tel qu'un rayonnement, ou d'un agent mutagène chimique, ou différentes concentrations de la Nitrosoguanidine puis à sélectionner dans un milieu approprié les mutants d'intérêt restants en utilisant leur spectre antibiotique.

Avantageusement, les souches mutantes obtenues présentent des capacités de production de certains métabolites, de 10 à 100 fois plus élevées que celles de la souche selon l'invention non mutée.

La souche beta-vulgaris I-3639 selon l'invention ou ses souches mutantes, montrent des activités biologiques importantes vis-à-vis de bactéries comme Micrococcus luteus, Bacillus substillus, Bacillus cereus et Streptomyces scabies, et contre des champignons phytopathogènes comme Fusarium oxysporum f.sp. albedinis, Botrytis cinerea, Phythium ultimum, Verticillium dahliae.

La souche Streptomyces beta-vulgaris selon l'invention ou ses souches mutantes présentent également une forte activité contre les champignons causant les maladies du bois de la vigne comme Phaeomoniella chlamydospora, Phaeomoniella aleophilum, Eutypa lata, Fomitiporia mediterranea et Botryosphaeria obtusa.

Le spectre d'activités de la souche beta-vulgaris selon l'invention vis-à-vis de certains micro-organismes est présenté dans le tableau ci-dessous. Quatre degrés d'activité ont été mesurés :
- pas d'activité
+ activité (inhibition de 10 à 15 mm)
++ forte activité (inhibition de 15 à 20 mm)
+++ très forte activité (inhibition supérieure à 20 mm)

| Micro-organisme | Activité de la souche selon l'invention |
|---|---|
| Phaeomoniella chlamydospora | ++ |
| Phaeomoniella aleophilum | ++ |
| Fomitiporia mediterranea | ++ |
| Eutypa lata | +++ |
| Botryosphaeria obtusa | +++ |
| Botryosphaeria dothidea | +++ |
| Sclerotium rolfsii | +++ |
| Botrytis cinerea | +++ |
| Verticillium dahliae | +++ |
| Fusarium oxysporum | ++ |
| Pythium ultimum | ++ |
| Streptomyces scabies | +++ |
| Micrococcus luteus | +++ |
| Bacillus substilis | +++ |
| Pseudomonas f luorescens | + |
| Escherichia coli | + |

### III. Filtrat de culture susceptible d'être produit par fermentation de la souche selon l'invention

Selon un autre aspect l'invention vise l'utilisation de la souche beta-vulgaris I-3639 ou d'une souche mutante pour l'obtention par fermentation dans un milieu liquide, d'une composition comprenant notamment des métabolites.

La souche beta-vulgaris selon l'invention peut en effet être utilisée pour produire par fermentation une composition, un filtrat de culture, contenant notamment des métabolites secondaires.

Le filtrat de culture est obtenu par culture de la souche beta-vulgaris I-3639 dans un milieu liquide dans un fermenteur.

Le procédé de production est le suivant :
- culture de bactéries beta-vulgaris selon l'invention ou une souche mutante dans un fermenteur à 30°C pendant 10 jours, dans un milieu liquide constitué de Glucose, d'extrait de Malte, d'extrait de levure et d'eau,
- centrifugation du mélange,
- filtration à travers un filtre avec des pores de 45µm de diamètre,
- récupération du filtrat de culture.

Le filtrat de culture est une composition comprenant des éléments du milieu de culture et les métabolites produits pendant la fermentation.

L'étape de fermentation peut être mise en oeuvre selon le protocole décrit en suivant.

Un prélèvement de la souche à partir d'une culture inclinée mature est inoculé dans un flacon Erlenmeyer contenant 50mL de milieu de culture stérile contenant 0,4% de glucose, 1% d'un extrait de malt et 0,4% d'un extrait de levure (ajusté au pH 7,2 avant stérilisation) et mis en culture sur un agitateur rotatif (250 tours par minute) à 30°C pendant 10 jours.

Les étapes de centrifugation et de filtration peuvent être mises en oeuvre selon le protocole décrit en suivant.

Le bouillon de culture (10 litres) est centrifugé à 12 000 tours par minute pendant 15 minutes, et le surnageant est extrait deux fois avec un volume égal d'acétate d'éthyle. La phase organique est déshydratée avec du Na₂SO₄ et séchée avec un évaporateur rotatif. L'extrait brut rouge obtenu est suspendu dans un volume minimum de MeOH, puis filtré à travers un filtre avec des pores de 45µm de diamètre.

Le filtrat de culture issu de la fermentation de la souche selon l'invention, peut être utilisé pour la lutte contre les agents phytopathogènes et/ou pour stimuler la croissance des plantes.

### IV. Composés actifs susceptibles d'être produits par fermentation de la souche selon l'invention

Selon un autre aspect l'invention vise l'utilisation de la souche beta-vulgaris I-3639 ou d'une souche mutante pour l'obtention par fermentation dans un milieu liquide, de composés actifs.

La souche beta-vulgaris selon l'invention peut en effet être utilisée pour produire par fermentation des métabolites secondaires, notamment des antibiotiques. Ces composés actifs peuvent être obtenus par :
- culture de la souche beta-vulgaris I-3639 ou d'une souche mutante dans un milieu liquide dans un fermenteur,
- centrifugation,
- filtration,
- puis purification.

Les étapes de fermentation, centrifugation et filtration sont telles que décrites au III.

La production de l'activité antibactérienne totale est réalisée sur une gélose nutritive par un test de diffusion sur gélose contre Bacillus subtilis ATCC 6633 et Mucore sp.. L'inhibition de la croissance est examinée après 24 heures d'incubation à 30°C. L'activité antimicrobienne est évaluée en mesurant le diamètre de la zone d'inhibition.

Pour récupérer les composés actifs produits par la souche selon l'invention ou une souche mutante et présents dans le filtrat de culture, l'étape de filtration est suivie d'une étape de purification des composés actifs, qui peut être réalisée comme indiqué ci-après.

Le filtrat est élué par HPLC en phase inverse dans les conditions suivantes pour obtenir des produits purs : colonne uptisphère UP5ODB C18 (250x7,8 mm) soumise à un système solvant à gradient linéaire de 0% à 100% de MeOH pendant 45 minutes et à gradient isocratique à 100% MeOH dans H₂O pendant 20 minutes, avec un débit de 1ml/minute et une détection UV à 220 nm. Les fractions correspondant aux pics actifs sont obtenues par test contre Bacillus subtilis et Mucore ramanniamus.

Les composés actifs obtenus sont notamment des métabolites secondaires tels que des antibiotiques impliqués dans la lutte biologique et le contrôle des maladies phytopathogènes.

### V. Utilisation d'une souche bete-vulgaris I-3639, d'une souche mutante, du filtrat de culture ou d'un composé actif

La souche selon l'invention présente des propriétés phytostimulatrices importantes si bien qu'elle peut être utilisée pour stimuler la croissance des plantes.

En particulier, la souche beta-vulgaris I-3639 est capable de stimuler la partie racinaire des plantes, en augmentant le nombre de poils absorbants racinaires, de stimuler la partie aérienne ainsi que la floraison des plantes. Elle est également capable d'améliorer signif icativement l'assimilation des nutriments par les plantes.

Il en est de même d'une souche mutante, du filtrat de culture ou des composés actifs obtenus par fermentation de la souche I-3639 ou d'une souche mutante.

En outre, les différentes études relatives aux caractéristiques de la souche ou d'une souche mutante, du filtrat de culture ou des composés actifs produits par cette souche, montrent que ladite souche (ou souche mutante), ledit filtrat et lesdits composés actifs sont très efficaces du point de vue de leurs propriétés antibactériennes et antifongiques, ce qui leur confèrent notamment la capacité d'être utilisés en tant qu'agents de lutte biologique.

La souche beta-vulgaris selon l'invention, une souche mutante, le filtrat de culture ou un composé actif produit à partir de cette souche, peuvent donc être utilisés en tant qu'agent antibactérien et/ou antifongique pour la protection des plantes et la lutte biologique contre des agents phytopathogènes.

Ils peuvent spécifiquement être utilisés pour la protection des plantes vis-à-vis de bactéries gram-positif ou encore vis-à-vis des champignons phytopathogènes. Des tests ont été effectués pour illustrer l'action de la souche selon l'invention, d'une souche mutante, du filtrat de culture et de ses composés actifs sur les plantes.

### 1) Etude des propriétés phytostimulatrices

Le but de cette étude est de montrer la capacité de la souche selon l'invention et de ses composés actifs, à stimuler la croissance des plantes.

L'étude est réalisée sur des plantules d'Arabidospsis thaliana.

Les plantules sont pré-traitées au niveau des racines par la souche selon l'invention, son filtrat de culture ou ses composés actifs obtenus après fermentation et purification, puis sont réensemencées sur un milieu de culture Gamgorg B5. L'ensemble est incubé dans une chambre de culture à 25°C.

La synthèse de la lecture des résultats effectuée après une semaine d'incubation, est représentée dans le tableau suivant :

| | Témoin | Traité avec la souche selon l'invention |
|---|---|---|
| Poids (g) | 0,140 | 0,205 |
| Taille des plants (mm) | 38 | 48 |
| Longueur des racines (mm) | 24 | 38 |

Ces résultats montrent bien que la souche I-3639 selon l'invention stimule clairement la croissance des plantes, notamment des plantules d'Arabidospsis thaliana.

En particulier elle stimule la partie racinaire des plantes. Elle permet également d'augmenter leur taille et leur poids de façon considérable.

La même étude a été réalisée sur des plantules de vitro-plants de vigne.

Les vitro-plants sont pré-traités au niveau des racines par la souche selon l'invention ou par le filtrat de culture ou un composé actif, puis sont réensemencés sur un milieu de culture Gamgorg B5. L'ensemble est incubé dans une chambre de culture à 25°C.

La lecture des résultats effectuée après une semaine d'incubation montre que la souche I-3639 stimule clairement la croissance des vitro-plants de la vigne, en particulier la partie racinaire.

### 2) Amélioration de l'assimilation des nutriments par les plantes

Cette étude a pour objectif d'évaluer l'effet de la souche I-3639, en particulier du filtrat de culture selon l'invention, sur l'assimilation des nutriments par les plantes.

Les essais sont menés sur le blé tendre.

Les graines de blé sont stérilisées en les plongeant dans un mélange d'eau distillée et de javel pendant 10 minutes. Elles sont ensuite rincées trois fois dans l'eau distillée stérile et séchées sur du papier Wattman sous une hotte.

Les graines sont ensuite plantées dans des pots en plastique perforés contenant chacun 500 g de terre. Chaque pot reçoit 10 graines plantées à 1 cm de profondeur. Les pots sont irrigués quotidiennement.

Après deux semaines de croissance, les feuilles sont traitées par pulvérisation foliaire avec le filtrat de culture selon l'invention (obtenu par la mise en oeuvre du procédé au point III) ou avec de l'eau (témoin) ou avec de l'engrais organominéral (témoin positif). Le filtrat est mélangé avec de l'eau. Différentes proportions ont été testées (30% et 50%).

Les tests sont répétés cinq fois pour chaque essai.

Après trois semaines de croissance, les feuilles sont broyées à 500 microns, puis séchées à l'étuve à 105°C jusqu'à poids constant.

L'assimilation de l'azote est mesurée par la méthode de Dumas. Pour les autres minéraux on effectue une analyse par spectrométrie d'émission atomique.

Les résultats obtenus sont présentés dans le tableau ci-dessous :

| | Azote | Phosphore | Potassium | Calcium | Magnésium | Bore | Cuivre | Fer | Manganèse | Zinc |
|---|---|---|---|---|---|---|---|---|---|---|
| Témoin (Eau) | 1,24 | 0,75 | 2,47 | 0,68 | 0,19 | 14 | 4,86 | 41,1 | 319 | 41,7 |
| Engrais organo-minéral | 1,74 | 0,54 | 2,27 | 0,74 | 0,25 | 17,3 | 4,63 | 232 | 1048 | 42,2 |
| Filtrat culture 30% | 1,83 | 0,63 | 2,47 | 0,8 | 0,27 | 15,5 | 8,36 | 387 | 1840 | 53,6 |
| Filtrat culture 50% | 1,63 | 0,58 | 2,35 | 0,75 | 0,24 | 14,6 | 8,48 | 359 | 1332 | 48,8 |

| | | | | | | | | | | |
|---|---|---|---|---|---|---|---|---|---|---|
| Les résultats pour l'Azote total, le Phosphore total, le Potassium total, le Calcium total et le Magnésium total sont donnés en pourcentage de matière sèche. Les résultats pour le Bore total, le Cuivre total, le Fer total, le Manganèse total, le Zinc total sont donnés en mg/kg de matière sèche. | | | | | | | | | | |

On constate que l'invention permet d'améliorer significativement l'assimilation des nutriments par les plantes et donc de favoriser leur croissance.

### 3) Etude des propriétés anti-fongiques et anti-bactériennes

### 3-a) Etude de la protection de la Vigne contre les maladies du bois

Cette étude a pour objectif de montrer la capacité de la souche selon l'invention, du filtrat de culture et de ses composés actifs à protéger in vitro des plants de vigne contre des maladies du bois (ESCA, Ethypiose, BDA) causées par des champignons phytopathogènes.

Le protocole opératoire est le suivant.

Des entre noeuds de vitro-plants de vigne (cépage sauvignon et cépage colombard) de 5 semaines sont découpés, traités avec des spores de la souche I-3639 puis ensemencés dans un milieu MS sous conditions contrôlées.

Après 5 semaines d'incubation, les feuilles de chaque vitro-plant sont traitées par une suspension de spores de champignons phytopathogène Eutypia lata.

La lecture des résultats est réalisée après une semaine d'infection.

Les résultats obtenus montrent que la souche I-3639 est capable de protéger la vigne contre les infections de champignons phytopathogènes.

### 3-b) Etude de la protection de la Vigne contre Botrytis cinerea

Cette étude a pour objectif de montrer la capacité de la souche selon l'invention à protéger des plants de vigne contre Botrytis cinerea, champignon phytopathogène responsable de la pourriture grise, une maladie cryptogamique qui sévit sur plusieurs cultures d'intérêt agronomique majeur comme la vigne, le tournesol ou la tomate.

Les essais sont menés sur des feuilles de plants de vigne au même stade de maturation. Les feuilles sont d'abord traitées par le champignon Botrytis cinerea pendant 24 heures, puis certaines sont traitées par pulvérisation foliaire du filtrat de culture ou de la souche selon l'invention.

Les résultats obtenus après 24 heures sont représentés sur les figures la (témoin sans traitement), 1b (feuilles traitées avec Botrytis cinerea puis la souche selon l'invention) et 1c (feuille traitées avec Botrytis cinerea). Les feuilles des plants de vigne mises en contact avec Botrytis cinerea puis traitées avec la souche selon l'invention (Figure 1b) sont plus vertes et ne présentent pas de tâches par rapport à celles non traitées (Figure 1c).

### 4) Activation des systèmes de défense naturels des plantes

L'objectif de cette étude est d'évaluer la capacité de la souche selon l'invention, du filtrat de culture et de ses composés actifs, à activer des gènes de défense naturelle des plantes, en particulier d'Arabidopsis thaliana.

Le protocole opératoire est décrit en suivant.

Les plantules d'Arabidopsis thaliana sont pré-traitées au niveau des racines par I-3639, une souche mutante ou par deux de ses composés actifs, puis sont réensemencées sur le milieu de culture Gamgorg B5 gélosé déjà inondé par une suspension de spores de Sclerotium rolfsii (10⁵ ufc/ml). L'ensemble est incubé dans une chambre de culture à 25°C.

La lecture des résultats est effectuée après une semaine.

On constate que le traitement de plantules d'Arabidopsis thaliana par la souche et par ses composés actifs, active l'expression des gènes liés à la défense des plantes.

L'expression des gènes de défense d'Arabidopsis thaliana est mesurée en utilisant la technique de la RT-PCR. Les gènes de défense des plantes étudiés sont PR1 (gène responsable de la synthèse des molécules antifongiques des plantes), PDF1.2a (gène responsable de l'activation des voie éthylène et jasmonate, deux voies importantes de la défense des plantes) et PALI.

La RT-PCR est réalisée sur des plantules cultivées pendant 10 jours. Elle est basée sur l'extraction de leur ARN qui est ensuite convertit en ADN complémentaire (ADNc) en utilisant le SuperscriptTM II RNAse H-reverse Transcriptase Kit (Invitrogen, Carisbad, USA) avec Oligo(dT)22.

Les échantillons d'ADNc obtenus sont amplifiés dans un mélange de produits de PCR.

Les amorces spécifiques utilisés sont :
- pour PR1 : PR1 f de séquence SEQ n°4 : 5' - CTG GCT ATT CTC GAT TTT TAA TCG - 3', PR1 r de séquence SEQ n°5 : 5' - TCC TGC ATA *TGA TGC TCC* TTA TTG - 3' ; excepté de produit de dimension de 562 pb,
- pour PDFl.2a : PDF1 f de séquence SEQ n°6: 5' - T*CA* T*GG C*T*A AG*T TT*G C*TT *CCA* T*CA* T*CA CCC -* 3', PDF1 r de séquence SEQ n°7 : - 5' *G*T*A GA*T TT*A ACA* T*GG GAC T*G*C GAC -* 3' ; excepté le produit de dimension de 252 pb,
- pour PAL1 : PAL1 f de séquence SEQ n°8 : 5' - *CGG AGG AGG AG*T *GGA CGC* TAT - 3', PAL1 r de séquence SEQ n°9 : 5' - TGC GAC ACC GTT TTT GGT TCT - 34 ; excepté le produit de dimension de 378 pb.

Le témoin de PCR est réalisé par l'utilisation du gène de nettoyage EF1 A4 (Liboz et al., 1990) : EF1 f de séquence SEQ n°10 : 5' - *A*T*G CCC CAG GAC A*T*C G*T*G* ATT TCA - 3', EF1 r de séquence SEQ n°11 : ' - TTG *GCG GCA CCC* TT*A GC*T *GGA* TCA - 3' ; excepté le produit de dimension de 708 pb.

Les conditions thermiques des cycles contiennent une étape de dénaturation initiale à 94°C 2 minutes, suivie par 34 cycles pour PR1, PDF1.2a et PAL1 ou par 26 cycles pour EF1 A4, de 94°C, 30 secondes, 52°C 30 secondes, 72°C 30 secondes et se termine à 72°c pendant 10 minutes.

Le produit PCR est chargé sur gel d'électrophorèse puis visualisé sous UV par fluorescence du bromure d'éthidium.

On constate que le traitement de plantules d'Arabidopsis thaliana par la souche et par ses composés actifs, active l'expression des gènes liés à la défense des plantes.

Les résultats d'expériences de la RT-PCR montrent que la souche beta-vulgaris selon l'invention active l'expression des gènes de défense des plantes PR1 (gène responsable de la synthèse des molécules antifongiques des plantes) et PDF1.2a (gène responsable de l'activation des voie éthylène et jasmonate, deux voies importantes de la défense des plantes).

L'activation des ces gènes PR1 et PDF1.2a par la souche selon l'invention conduit à la production :
- d'antibiotiques végétaux, les phytoalexines,
- de protéines de défense, les PR-protéines, et
- de composés destinés à renforcer les parois des cellules végétales.

## Revendications

1. Souche actinomycète Streptomyces beta-vulgaris présentant une séquence de l'ADNr 16S SEQ n°1, ladite souche étant déposée à la CNCM sous le numéro 1-3639 ou obtenue par mutation sélective à partir de la souche déposée à la CNCM sous le numéro 1-3639,
ladite souche étant efficace pour :
- stimuler la croissance des plantes, et/ou
- lutter contre des agents phytopathogènes, et/ou
- stimuler les défenses naturelles des plantes, et/ou
- améliorer l'assimilation des nutriments par des plantes.

2. Utilisation d'une souche selon la revendication 1, pour l'obtention d'un filtrat de culture par fermentation dans un milieu liquide de ladite souche, centrifugation et filtration.

3. Utilisation de la souche selon la revendication 1, pour l'obtention de métabolites secondaires par fermentation dans un milieu liquide de ladite souche.

4. Utilisation d'une souche selon la revendication 1 ou d'un filtrat de culture obtenu selon la revendication 2, pour stimuler la croissance des plantes.

5. Utilisation d'une souche selon la revendication 1, d'un filtrat de culture obtenu selon la revendication 2, pour stimuler la partie racinaire des plantes.

6. Utilisation d'une souche selon la revendication 1, d'un filtrat de culture obtenu selon la revendication 2, en tant qu'agent antibactérien pour la protection des plantes et la lutte biologique contre des agents phytopathogènes.

7. Utilisation selon la revendication 6, pour la protection des plantes vis-à-vis des bactéries gram-positif.

8. Utilisation d'une souche selon la revendication 1, d'un filtrat de culture obtenu selon la revendication 2, en tant qu'agent antifongique pour la protection des plantes et la lutte biologique contre des agents phytopathogènes.

9. Utilisation d'une souche selon la revendication 8, pour la protection des plantes vis-à-vis de Botrytis cinerea,.

10. Utilisation d'une souche selon la revendication 1, d'un filtrat de culture obtenu selon la revendication 2, pour activer les systèmes de défense naturelle des plantes.

11. Utilisation selon la revendication 10, pour stimuler l'expression des gènes de défense des plantes PR1 et PDF1.2a.

12. Utilisation d'une souche selon la revendication 1, d'un filtrat de culture obtenu selon la revendication 2, pour améliorer l'assimilation des nutriments par les plantes.

13. Utilisation selon la revendication 12, pour améliorer l'assimilation par les plantes du Potassium, du Zinc, du Bore, du Calcium, du Cuivre, du Phosphore, du Magnésium, du Fer, du Manganèse et de l'azote.

## Patentansprüche

1. Actinomycetstamm Streptomyces beta-vulgaris mit einer Sequenz der 16S rRNA SEQ Nr. 1, wobei der Stamm bei der CNCM unter der Nummer I-3639 hinterlegt ist oder durch eine selektive Mutation auf der Grundlage des bei der CNCM unter der Nummer I-3639 hinterlegten Stamms erhalten wird,
wobei der Stamm wirksam ist, um:
- das Wachstum der Pflanzen zu stimulieren, und/oder
- gegen phytopathogene Mittel zu kämpfen, und/oder
- die natürliche Abwehr der Pflanzen zu stimulieren, und/oder
- die Assimilierung der Nährstoffe durch die Pflanzen zu verbessern.

2. Verwendung eines Stamms nach Anspruch 1 zum Erhalt eines Kulturfiltrats durch Gärung in einem flüssigen Medium des Stamms, Zentrifugieren und Filtern.

3. Verwendung des Stamms nach Anspruch 1 zum Erhalt von sekundären Metaboliten durch Gärung in einem flüssigen Medium des Stamms.

4. Verwendung eines Stamms nach Anspruch 1 oder eines Kulturfiltrats, erhalten nach Anspruch 2, um das Wachstum der Pflanzen zu stimulieren.

5. Verwendung eines Stamms nach Anspruch 1 eines Kulturfiltrats, erhalten nach Anspruch 2, um den Wurzelteil der Pflanzen zu stimulieren.

6. Verwendung eines Stamms nach Anspruch 1 eines Kulturfiltrats, erhalten nach Anspruch 2, als antibakterielles Mittel zum Schutz der Pflanzen und zum biologischen Kampf gegen phytopathogene Mittel.

7. Verwendung nach Anspruch 6 zum Schutz der Pflanzen gegen grampositive Bakterien.

8. Verwendung eines Stamms nach Anspruch 1 eines Kulturfiltrats, erhalten nach Anspruch 2, als antimykotisches Mittel zum Schutz der Pflanzen und zum biologischen Kampf gegen phytopathogene Mittel.

9. Verwendung eines Stamms nach Anspruch 8 zum Schutz der Pflanzen gegen Botrytis cinerea.

10. Verwendung eines Stamms nach Anspruch 1 eines Kulturfiltrats, erhalten nach Anspruch 2, um die natürlichen Abwehrsysteme der Pflanzen zu aktivieren.

11. Verwendung nach Anspruch 10, um die Expression der Abwehrgene der Pflanzen PR1 und PDF1.2a zu stimulieren.

12. Verwendung eines Stamms nach Anspruch 1 eines Kulturfiltrats, erhalten nach Anspruch 2, um die Assimilierung der Nährstoffe durch die Pflanzen zu verbessern.

13. Verwendung nach Anspruch 12, um die Assimilierung durch die Pflanzen von Kalium, Zink, Bor, Kalzium, Kupfer, Phosphor, Magnesium, Eisen, Mangan und Stickstoff zu verbessern.

## Claims

1. Actinomycete Streptomyces beta-vulgaris strain having a sequence of rDNA 16S SEQ No. 1, said strain being deposited at the CNCM under number I-3639 or obtained by selective mutation from the strain deposited at the CNCM under number I-3639,
said strain being efficient for:
- stimulating the growth of plants, and/or
- struggling against the phytopathogenic agents, and/or
- stimulating the natural defenses of plants, and/or
- improving the assimilation of nutrients by the plants.

2. Use of the strain according to Claim 1 for obtaining a culture filtrate by fermentation in a liquid medium of said strain, centrifugation and filtration.

3. Use of the strain according to Claim 1 for obtaining active compounds by fermentation in a liquid medium of said strain.

4. Use of a strain according to Claim 1 or a culture filtrate that is obtained according to Claim 2 for stimulating the growth of plants.

5. Use of the strain according to Claim 1, a culture filtrate that is obtained according to Claim 2 for stimulating the root portion of the plants.

6. Use of a strain according to Claim 1, a culture filtrate that is obtained according to Claim 2 as an antibacterial agent for the protection of plants and the biological struggle against phytopathogenic agents.

7. Use according to Claim 6, for the protection of plants relative to gram-positive bacteria.

8. Use of a strain according to Claim 1, a culture filtrate that is obtained according to Claim 2 as an antifungal agent for the protection of plants and the biological struggle against phytopathogenic agents.

9. Use of a strain according to Claim 8 for the protection of plants relative to Botrytis cinerea.

10. Use of a strain according to Claim 1, a culture filtrate that is obtained according to Claim 2 for activating the natural defense systems of plants.

11. Use according to Claim 10 for stimulating the expression of the defense genes of plants PR1 and PDF1.2a.

12. Use of a strain according to Claim 1, a culture filtrate that is obtained according to Claim 2 for improving the assimilation of nutrients by the plants.

13. Use according to Claim 12 for improving the assimilation by the plants of potassium, zinc, boron, calcium, copper, phosphorus, magnesium, iron, manganese, and nitrogen.
